# EUROPEAN PATENT APPLICATION

(11) **EP 3 539 467 A1**
(43) Date of publication of application: **18.09.2019**
(21) Application number: 17874854.7
(22) Date of filing: 23.11.2017
(51) Int. Cl.: A61B 5/021, A61B 5/00

(54) **WRIST BLOOD PRESSURE MONITOR**

(30) Priority: 23.11.2016 KR 20160156362
(71) Applicant: Charmcare Co., Ltd., Geumcheon-gu, Seoul 08591 (KR)
(72) Inventor: LEE, Dong Hwa, Yongin-si Gyeonggi-do 16895 (KR)
(74) Representative: Manitz Finsterwald Patent- und Rechtsanwaltspartnerschaft mbB
(86) International application number: PCT/KR2017/013418
(87) International publication number: WO 2018/097617

(57) **Abstract**

Disclosed is a wrist blood pressure monitor wearable on a wrist. One aspect of the present invention provides a wrist blood pressure monitor comprising: a wrist cuff having a wrist strap; and a blood pressure measuring sensor disposed on the wrist cuff, wherein the wrist strap comprises a strap frame which is arranged around the ulna along the circumferential direction of the wrist and is bent in a predetermined shape so as to cross a portion where the ulnar artery passes, so as to maintain the shape of the skeleton of the strap frame, and a connecting strap disposed on the strap frame and connecting one end and the other end of the strap frame. The present invention allows a user to conveniently measure blood pressure, and ensure the blood flow of the ulnar artery while the blood pressure measurement is performed, thereby improving the accuracy.

## Description

### [Technical Field]

The present invention relates to a wrist blood pressure monitor which is wearable on a wrist to measure blood pressure, and more particularly to a wrist blood pressure monitor which may ensure blood flow of the ulnar artery while blood pressure measurement is performed.

### [Background Art]

In general, the pressure of blood applied to the walls of blood vessels is referred to as blood pressure, and the heart repeats contraction and expansion about 60 to 80 times per minute. The pressure of blood applied to the walls of blood vessels when the heart contracts and thus expels blood is referred to as 'systolic pressure', and is highest and thus is also referred to as 'maximum blood pressure'. Further, the pressure of blood applied to the walls of blood vessels when the heart expands and thus receives blood is referred to as 'diastolic pressure', and is lowest and thus is also referred to as 'minimum blood pressure'.

In general, a normal person has systolic pressure of 120 mmHg and diastolic pressure of 80 mmHg. One in four Korean adults corresponds to hypertensive, the rate of hypertensive tends to rapidly increase after the age of 40, and some patients are classified as hypotensive.

The reason why hypertension becomes an issue is that, if hypertension is not properly managed and is neglected, hypertension may cause other complications which may threaten patient's life, such as eye disease, renal disease, artery disease, brain disease and heart disease. Therefore, a patient at risk of complications or having complications requires continuous blood pressure measurement and management.

As interest in adult diseases, such as hypertension, and health increases, various kinds of blood pressure measurement apparatuses are being developed.

Blood pressure measurement methods include a Korotkoff sounds method, an oscillometric method and a tonometric method.

The Korotkoff sounds method is a typical blood pressure measurement method, in which, during a process of blocking blood flow by applying sufficient pressure to a body part through which arterial blood passes and then lowering the pressure, pressure at a moment when a sound of pulse is initially heard is measured as systolic pressure, and pressure at a moment when the sound of pulse disappears is measured as diastolic pressure.

The oscillometric method and the tonometric method are applied to digital blood pressure measurement apparatuses.

In the oscillometric method, a pulse wave, generated during a process of applying sufficient pressure to a body part through which arterial blood passes to block blood flow and then lowering the pressure at a designated speed, in the same manner as in the Korotkoff sounds method, or then raising the pressure applied to the body part at a designated speed, is sensed and, thus, systolic pressure and diastolic pressure are measured.

Here, pressure when the amplitude of the pulse wave reaches a designated level, as compared to the maximum amplitude, may be measured as systolic pressure or diastolic pressure, or pressure when the rate of change of the amplitude of the pulse wave is rapidly changed may be measured as systolic pressure or diastolic pressure.

Further, during the process of lowering the applied pressure at the designated speed after application of pressure, systolic pressure is measured before the moment when the amplitude of the pulse wave reaches the maximum amplitude, and diastolic pressure is measured after the moment when the amplitude of the pulse wave reaches the maximum amplitude. In contrast, during the process of raising the applied pressure at the designated speed, systolic pressure is measured after the moment when the amplitude of the pulse wave reaches the maximum amplitude, and diastolic pressure is measured before the moment when the amplitude of the pulse wave reaches the maximum amplitude.

Next, in the tonometric method, a designated pressure having a magnitude which does not completely block arterial blood flow is applied to a body part, and blood pressure is continuously measured using the size and shape of a pulse wave generated at this time.

Sphygmomanometers to measure blood pressure using the above-described methods are the most basic medical apparatuses to measure blood pressure serving as a basic health index, are essentially provided in general hospitals, and are frequently used in homes or sports centers so as to measure blood pressures of individuals.

However, in case of the conventional sphygmomanometers, forearm wearable products, which are wound on a forearm (an upper part, i.e., a portion from a shoulder to an elbow) to measure blood pressure whenever blood pressure measurement is necessary, are on the market, and such products are difficulty to carry about and cannot readily measure blood pressure when a user wants.

For example, the conventional oscillometric-type sphygmomanometer requires a main body wound on a wrist or a forearm to press the wrist or the forearm, a pressure cuff, a tube plug, an air hose, etc. and thus have a complicated configuration and a large volume, and, when used, for the purpose of correct measurement, there is a lot of preparation, i.e., the air hose should be inserted into the main body so as to face down, the cuff should be worn on a region located at a position spaced upwardly apart from an elbow by a distance of 1-2 cm, at a measurement preparation stage, and thus the conventional oscillometric-type sphygmomanometer is inconvenient to use.

Particularly, since a degree of change in blood pressure is different according to physical characteristics of a subject, patients having complications need to measure their blood pressure periodically and/or immediately whenever they feel abnormalities in their bodies, and a mercury sphygmomanometer and an electronic sphygmomanometer provide inconvenience to the patients due to problems which will be described below.

First, the mercury sphygmomanometer and the electronic sphygmomanometer use a cuff requiring air injection, and the cuff has a large volume and thus it is inconvenient for a user to wear the cuff in real time.

Further, the mercury sphygmomanometer using the above air pressure cuff has excessively large volume and weight for a patient who should frequently measure blood pressure, is inconvenient for a subject (the patient) to carry about, and causes cumbersomeness because the subject wears the mercury sphygmomanometer whenever the subject measures blood pressure.

Further, a pneumatic electronic sphygmomanometer having the same precision as the above-described conventional sphygmomanometer is provided, but, if the pneumatic electronic sphygmomanometer is applied to a case that a patient should continually carry the pneumatic electronic sphygmomanometer to periodically measure blood pressure, the pneumatic electronic sphygmomanometer using an electronic pump and an air injection type cuff has a heavy weight and a large volume and thus causes patient mobility difficulty.

In addition, there are non-pressure type electronic sphygmomanometers which measure blood pressure based on a pulse at a wrist or a fingertip and various parameters, but, since it is difficult to precisely specify these parameters and universally provide the parameters, the non-pressure type electronic sphygmomanometers have low precision and are difficult to apply to patients who should periodically measure precise blood pressure.

Recently, a sphygmomanometer which is wearable on a wrist like a wristwatch so as to measure blood pressure is being developed under the name of a wrist sphygmomanometer, a wrist wearable sphygmomanometer or a wristwatch type sphygmomanometer.

However, the conventional wrist sphygmomanometer is simply worn on a wrist to measure blood pressure and thus is referred to as a wristwatch type sphygmomanometer, but is provided with a mechanical/electronic pumping device, i.e., an air pump, to press the wrist and thus has problems, such as a complicated structure, difficulty in manipulation and operation, easy occurrence of failure, high manufacturing costs, etc. Further, in the conventional wrist sphygmomanometer, air should be injected into an air bag to press the wrist whenever blood pressure is measured, so as to sufficiently press a region of the wrist through which the radial artery passes, precision in measurement of blood pressure may be lowered due to poor adhesion of the air bag to the surface of the wrist and, when the wrist is pressed, blood flow in the ulnar artery together with the radial artery may be blocked.

### [Disclosure]

### [Technical Problem]

Therefore, the present invention has been made in view of the above problems, and it is an object of the present invention to provide a wrist blood pressure monitor which may ensure blood flow of the ulnar artery while blood pressure measurement is performed by pressing a wrist.

It is another object of the present invention to provide a wrist blood pressure monitor with the air bag which enables self-air supply.

### [Technical Solution]

In accordance with the present invention, the above and other objects can be accomplished by the provision of a wrist blood pressure monitor wearable on a wrist, including a wrist cuff including a wrist strap to be worn on the wrist, and a blood pressure measurement sensor provided on the wrist cuff, wherein the wrist strap includes a strap frame arranged around the ulna in a circumferential direction of the wrist and is bent in a predetermined shape to cross a portion of the wrist through which the ulnar artery passes, so as to maintain a shape of a skeleton of the strap frame, and a connection strap provided on the strap frame and connecting one end and the other end of the strap frame.

The wrist cuff may further include an air bag provided on the wrist strap to press a portion of the wrist through which the radial artery passes.

A valve may be connected to the air bag so as to enable air supply. Further, the valve may be a check valve configured to be opened due to a pressure difference between an air pressure inside the air bag and atmospheric pressure outside the air bag, so as to implement self-air supply of the air bag.

The blood pressure measurement sensor may be provided at the air bag, without being limited thereto. The air bag may be provided on the connection strap so as to press the portion of the wrist through which the radial artery passes.

The connection strap may extend from one end of the strap frame and be detachably connected to the other end of the strap frame. The wrist cuff may have a closed loop shape in which the entirety of the wrist cuff is continuously connected without any intermittent part, for example, an integral loop shape without any intermittent part (a part which is cut and then connected).

The connection strap may include a main strap provided with an air bag to press the wrist, and extending from one end of the strap frame, and an assistant strap extending from the main strap and detachably connected to the other end of the strap frame.

The strap frame may include a base frame configured to support the connection strap, and an assistant frame movably provided on the base frame, so as to adjust a height of the wrist cuff corresponding to a wrist thickness.

The wrist cuff may have a sealed-type closed loop shape being continuously connected.

### [Advantageous effects]

A wrist blood pressure monitor in accordance with one embodiment of the present invention has the following advantages.

First, the blood pressure monitor in accordance with the embodiment of the present invention allows a user to conveniently measure blood pressure whenever blood pressure measurement is necessary, and ensures blood flow of the ulnar artery while the blood pressure measurement is performed by pressing a wrist, thus increasing accuracy of blood pressure measurement.

Further, the blood pressure monitor in accordance with the embodiment of the present invention may achieve size (height) adjustment corresponding to a user's wrist thickness while maintaining a basic skeleton, and achieve self-air supplement (self-air supply) of an air bag corresponding to change in external environments, for example, temperature change, thus preventing degradation of performance of the wrist blood pressure monitor.

In addition, in order to detect change in blood pressure, it is important to periodically measure blood pressure at the same time every day and record the measured blood pressure value, and the blood pressure monitor in accordance with the embodiment of the present invention may automatically measure blood pressure at a designated time and, particularly, if an alarm is provided, is advantageous with respect to regular blood pressure measurement without missing of the blood pressure measuring time.

Moreover, the blood pressure monitor in accordance with the embodiment of the present invention may transmit measured blood pressure data to a family doctor or a medical specialist through a wireless communication unit to be used in analysis, thus achieving management, particularly, effective blood pressure management of patients suffering hypertension, diabetes, hepatosis, arteriosclerosis, peripheral nerve disorder of blood circulation, etc.

### [Description of Drawings]

The above and other objects, features and other advantages of the present invention will be more clearly understood from the following detailed description taken in conjunction with the accompanying drawings, in which:
FIG. 1 is a side view schematically illustrating a wrist blood pressure monitor in accordance with one embodiment of the present invention;
FIG. 2 is a cross-sectional view illustrating a wrist cuff of the wrist blood pressure monitor shown in FIG. 1;
FIG. 3 is a side view illustrating a state in which the wrist blood pressure monitor forms a closed loop;
FIG. 4 is a side view schematically illustrating a wrist blood pressure monitor in accordance with another embodiment of the present invention; and
FIG. 5 is a block diagram illustrating the overall configuration of the wrist blood pressure monitor in accordance with the present invention.

### [Best Mode]

Reference will now be made in detail to the exemplary embodiments of the present invention, examples of which are illustrated in the accompanying drawings. Wherever possible, the same reference numbers will be used throughout the drawings to refer to the same or like parts.

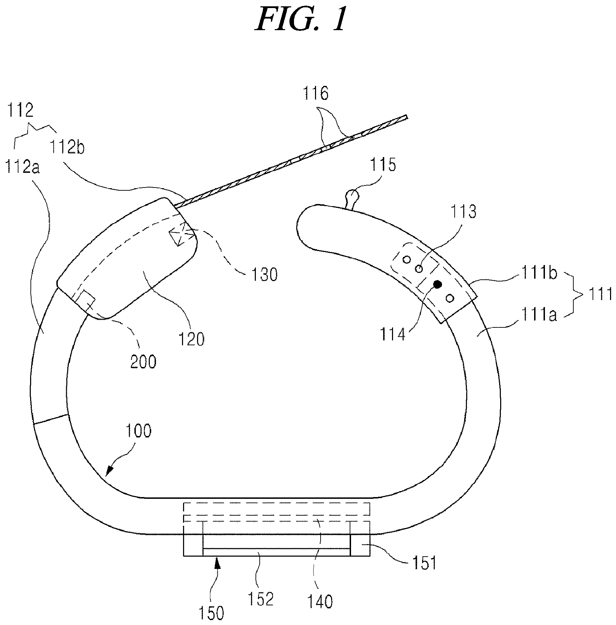

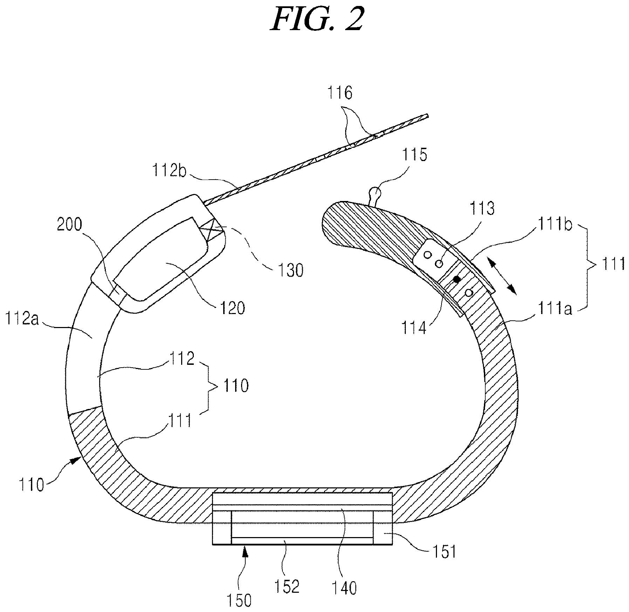

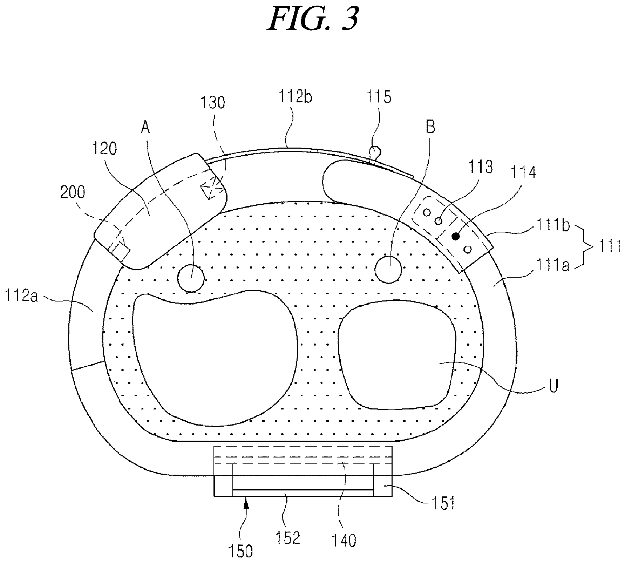

First, referring to FIGS. 1 to 3, a wrist blood pressure monitor in accordance with one embodiment of the present invention will be described. FIG. 1 is a side view schematically illustrating the wrist blood pressure monitor in accordance with one embodiment of the present invention, FIG. 2 is a cross-sectional view illustrating a wrist cuff of the wrist blood pressure monitor shown in FIG. 1, and FIG. 3 is a side view illustrating a state in which the wrist blood pressure monitor forms a closed loop.

Referring to FIGS. 1 to 3, the wrist blood pressure monitor in accordance with this embodiment is wearable on a wrist, and includes a wrist cuff 100 wearable on the wrist and a blood pressure measurement sensor 200.

The wrist cuff 100 is wearable on the wrist like a wristwatch, has a wrist strap 110 to be worn on the wrist, and the above-described blood pressure measurement sensor 200 is provided on the wrist cuff 100.

Although the wrist cuff 100 may have an air bag-less structure, the wrist cuff 100 in accordance with this embodiment may further include an air bag 120 to press a designated region of the wrist, for example, a region of the wrist through which the radial artery A passes, and, in this embodiment, the air bag 120 is provided on the wrist strap 110 and the sensor 200 is connected to the air bag 120.

That is to say, the wrist blood pressure monitor in accordance with this embodiment is a blood pressure measurement cuff which surrounds the wrist to measure blood pressure, and may be provided as a portable type which a user may carry during activity like a wristwatch, be worn on the wrist of a bedridden patient having difficulty moving around so as to frequently check blood pressure, and be connected to other apparatuses, for example, a blood pressure management monitor, by wire or wirelessly.

The wrist strap 110 is a band or belt type which is wound on a wrist, the air bag 120 is provided on the wrist strap 110 to press the wrist, and, in this embodiment, the sensor 200 is provided on the air bag 120 to sense change in pressure of the air bag 120, but the position of the sensor is not limited thereto and the sensor may be provided on an air path communicating with the inside of the air bag.

Although the air bag 120, which presses the wrist to measure blood pressure, may have a structure to prevent air injection, i.e., a sealed structure which may not be opened and closed, in this embodiment, the air bag 120 may be configured as a type in which air injection, i.e., air supply, is possible, for example, a type to which a valve 130 is connected.

In this embodiment, the valve 130 implements self-air supply of the air bag. A check valve to achieve air flow in one direction may be applied as the valve 130, and, as the valve 130 is opened due to a pressure difference between the inside of the air bag and the outside of the air bag, self-air supply of the air bag 120 is achieved.

Although the valve 130 may be connected to the air bag 120 by the air path, such as a tube or the like, in this embodiment, the valve 130 is provided on the air bag 120. Therefore, in this embodiment, the sensor 200 and the valve 130 are installed on the air bag 120 itself, and, if the internal pressure of the air bag 120 is lower than the external pressure, i.e., atmospheric pressure, the air bag 120 may be supplemented with air through the valve 130.

The air bag 120 may be an elastic air bag having self-restoring force, for example, be formed of an elastic material, such as silicone, urethane or rubber, so as to be restored to an initial shape thereof having a predetermined volume. The air bag 120 presses the surface of the wrist, for example, a region of the wrist through which the radial artery A passes, and, in this embodiment, a device to sense pressure of the air bag 120, for example, a pressure sensor, may be applied as the sensor 200.

Various sensors which may measure air pressure may be applied as the sensor 200, and, in this embodiment, the sensor 200 is connected to a controller, i.e., a circuit board 140 which calculates blood pressure, through a cable (not shown) installed on the wrist strap 110.

Further, the wrist cuff 100 may be formed to have a structure in which a specific portion of the wrist cuff 100 is intermittent (a structure which may be cut and then connected - a detachable structure), or be formed to have a sealed-type closed loop shape in which the entirety of the cuff is continuously connected without any intermittent part, for example, an integral loop shape without any intermittent part (a part which is cut and then connected).

The wrist cuff 100 is divided into a left strap and a right strap based on a display unit 150 that outputs blood pressure to the outside. Of course, the wrist cuff 100 may have the above-described integral closed loop shape provided with no detachable connection part (intermittent part), i.e., a continuously connected loop shape having a circulation structure, particularly an elastically expandable loop shape.

The wrist strap 110 includes a strap frame 111 and a connection strap 112. The strap frame 111 is a frame element, which is bent into a designated shape and worn on a predetermined portion of the wrist so as to be wound on a portion of the wrist, particularly a portion of the wrist through which the ulna passes, and forms the skeleton of the wrist cuff 100. Of course, the strap frame 111 may have elasticity of a designated level which resists bending force.

That is, the strap frame 111 is bent into the designated shape to maintain the shape of the framework of the wrist strap, and is disposed around the ulna along the circumferential direction of the wrist to cross the portion of the wrist through which the ulnar artery B passes.

The strap frame 111 may be formed of a hard material having shape stability of a designated level or more, for example, metal, plastic, tough rubber, or the like, so as to maintain a constant shape. Further, the connection strap 112 is provided on the strap frame 111. More specifically, the connection strap 112 connects one end and the other end of the strap frame 111.

Although, in this embodiment, one end of the connection strap 112 is integrally connected to one end of the strap frame 111 and the other end of the connection strap 112 is detachably connected to the other end of the strap frame 111, the other end of the connection strap 112 may be integrally fixed to the other end of the strap frame 111 (so as not to be intermittent) and thus form the wrist cuff having the above-described closed loop structure, i.e., the sealed-type wrist cuff.

In more detail, the connection strap 112 in this embodiment extends from one end of the strap frame 111 and is detachably connected to the other end of the strap frame 111. The connection strap 112 may be a flexible strap supported by the strap frame 111, i.e., a strap formed of a flexible material which is easily bendable, for example, rubber, silicone, fabric or leather, and form a section connecting one end and the other end of the strap frame 111.

Of course, at least a part of the strap frame 111 may be provided inside the connection strap 112 and thus be covered with the connection strap 112.

In this embodiment, the air bag 120 is provided on the connection strap 112, and presses the region of the wrist through which the radial artery A passes when blood pressure is measured. Further, the strap frame 111 is disposed along the circumference of the ulna and has a shape having curvature so as to cover the region of the wrist through which the ulnar artery B passes.

Therefore, in accordance with this embodiment, the strap frame 111 covers the region of the wrist through which the ulnar artery B passes to protect the ulnar artery B, and thus ensures blood flow of the ulnar artery B (i.e., prevents blocking of the blood flow) during measurement of blood pressure, and prevents blood flow of the ulnar artery from being completely blocked by pressing using the wrist cuff, thereby performing measurement of blood pressure through the tonometric method. That is, the strap frame 111 serves as a frame to protect the ulnar artery B so that blood flow in the ulnar artery B continues during measurement of blood pressure.

Further, the strap frame 111 may include a base frame 111a and an assistant frame 111b. The base frame 111a is fixed to the connection frame and supports the connection strap 112.

The assistant frame 111b is a height adjusting frame which is movably provided on the base frame 111a, to adjust a height of the wrist cuff 100 corresponding to a wrist thickness. Therefore, the strap frame 111 forms a skeleton having a length adjustable structure.

The base frame 111a and the assistant frame 111b may be manufactured of the same material or be manufactured of different materials, but they are manufactured to have designated rigidity so as to maintain the shape of the skeleton of the wrist cuff 100.

Referring to FIGS. 1 to 3, the strap frame 111 has a bent shape, for example, is bent at an obtuse angle or bent to have curvature suitable for the surface of the wrist around the ulna, and the assistant frame 111b is assembled with the base frame 111a to be movable in a length direction of the strap frame 111.

Although the assistant frame 111b may be inserted into the base frame 111a so as to be slidable, in this embodiment, the base frame 111a is inserted into the assistant frame 111b.

A plurality of assembly holes 113 to adjust the height of the wrist cuff is formed through the assistant frame 111b by intervals in a length direction of the assistant frame 111b, at least one assembly pin 114 is provided on the base frame 111a and is elastically supported in the outward direction of the base frame 111a by an elastic member (not shown), such as a spring, installed in the base frame 111a, and, in this embodiment, the assembly pin 114 is elastically installed at an end portion of the base frame 111a, particularly, a portion of the base frame 111a which is inserted into the assistant frame 111b. A position adjusting structure using a pin and holes corresponds to well-known technology and a detailed description thereof will thus be omitted.

Therefore, the assembly pin 114 is coupled to any one of the assembly holes 113, and the height of the wrist cuff 100, more particularly, the height of the strap frame 111 may be adjusted according to coupled positions of the assembly pin 114.

The wrist cuff 100 may further include the display unit 150 to display blood pressure of a user (a subject), i.e., a wearer, and the above-described circuit board 140 is built in the display unit 150.

The display unit 150 may have a blood pressure display function, and further have a clock function. For example, the display unit 150 may be switched between a blood pressure output mode and a clock mode, or the display unit 150 may simultaneously display both blood pressure and time.

In this embodiment, the display unit 150 includes a case 151 to receive electronic parts and a display window 152 provided on the case 151, and the case 151 is mounted on the above-described wrist strap 110. The display window 152 displays blood pressure, and further, may display time.

The circuit board 140 is electrically connected to the sensor 200, calculates blood pressure and further outputs a blood pressure value to the display window 152.

Although not shown in the drawings, a battery to supply power to the wrist blood pressure monitor may be installed in the case 151. Of course, the wrist blood pressure monitor in accordance with this embodiment may receive power from the outside through a power cable, or be operated based on sunlight.

Further, the display unit may have an alarm function, for example, an alarm function of informing of a blood pressure checking time and/or an alarm function of informing that checking of blood pressure has been normally completed. Thereby, the user may precisely measure/record blood pressure at a time when checking of blood pressure is needed. In this embodiment, the display unit 150 is mounted on the strap frame 111.

One side of the wrist cuff 100 (the other end of the connection strap) and the other end of the wrist cuff 100 (the other end of the above-described strap frame) are detachably connected by a detachable unit, such as a Velcro strap, a hook, a button or a buckle, and thereby the wrist cuff 100 forms an intermittent loop shape. Of course, the wrist cuff 100 may form a closed loop structure in which the wrist cuff 100 is continuously connected, i.e., an integral band structure, without any intermittent part, as described above.

The connection strap 112 includes a main strap 112a and an assistant strap 112b, so that the wrist cuff 100 has the intermittent loop shape.

The main strap 112a extends from one end of the strap frame 111, and, in this embodiment, the air bag 120 is provided on the main strap 112a. The assistant strap 112b extends from the main strap 112a, and is detachably connected to the other end of the strap frame 111b, more particularly, to the assistant frame 111b.

In this embodiment, the assistant strap 112b is formed of a flexible member, such as a thin string, strap or band, at least one strap connection hole 116 is formed through the assistant strap 112b, and a fixing protrusion 115, such as a pin or a hook, which is inserted into the strap connection hole 116, is provided on the strap frame, particularly, the assistant frame 111b. In order to stably maintain connection of the assistant strap 112b, an enlarged head having a greater diameter than the diameter of the strap connection hole 116 is formed at the top of the fixing protrusion 115. At least a part of the connection strap 112 may have elasticity. For example, the assistant strap 112b may be an elastic strap, for example, a strap formed of an elastic material, such as rubber or silicone.

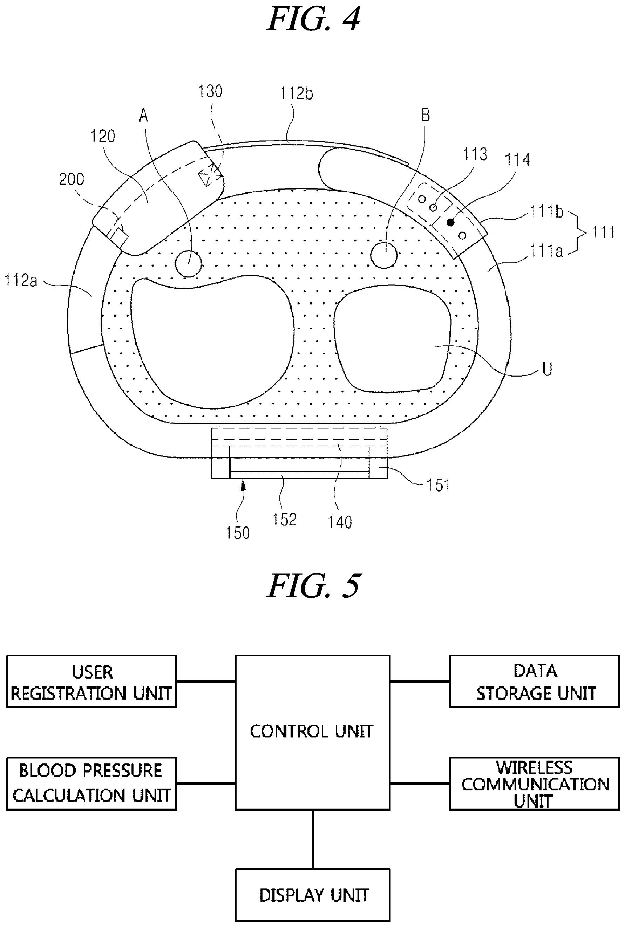

As exemplarily shown in FIG. 4, the connection strap 112 may form a section to integrally connect both ends of the strap frame 111 and thus form the sealed-type closed loop structure without any intermittent part, as described above, and, in this case, the connection strap 112 may have elasticity to be stretchable in the length direction thereof. That is, both ends of the connection strap 112 are integrally connected to both ends of the strap frame 111 so as not to be intermittent. In more detail, for example, the end portion of the assistant strap 112b may be integrally connected to the strap frame (assistant frame) by a bonding method. Further, at least a section of the entirety of the connection strap 112, for example, the assistant strap 112b may have elasticity.

Referring to FIG. 5, the wrist blood pressure monitor in this embodiment may further include a wireless communication unit using a Bluetooth scheme or the like to transmit blood pressure data (user's blood pressure) to a designated terminal, for example, a smartphone, other terminals or a management module, in addition to the display unit.

The wireless communication unit may be configured to transmit a specific signal in a specific situation, for example, an emergency rescue signal in an emergency situation, to external communication equipment, in addition to the blood pressure data. Therefore, real-time blood pressure data of the subject (user) may be transmitted to a manager, such as a family doctor or a medical specialist, through the smartphone, and be used in stable health management of the subject.

That is, persons suffering hypertension, diabetes, hepatosis, arteriosclerosis, peripheral nerve disorder of blood circulation, etc. require more careful attention, and the wrist blood pressure monitor in this embodiment has the data transmission function and may thus achieve management, particularly, effective blood pressure management of these patients.

Further, the wrist blood pressure monitor may include a blood pressure calculation unit to calculate blood pressure of the subject based on a signal from the pressure sensor, a data store unit to storage blood pressure data, a user registration unit to manage measurement histories of users and manage user IDs, and a control unit to collect and analyze data and thus to transmit a designated emergency rescue signal if a user is in an emergency state.

Although the exemplary embodiments of the present invention have been disclosed for illustrative purposes, those skilled in the art will appreciate that various modifications, additions and substitutions are possible, without departing from the scope and spirit of the invention as disclosed in the accompanying claims.

Therefore, the above-described embodiments are not limited and are exemplary, and thus the present invention is not limited to the above description and may be modified within the spirit of the invention, the scope of which is defined in the accompanying claims and their equivalents.

### [industrial Applicability]

As is apparent from the above description, the present invention provides a portable wrist blood pressure monitor, which allows a user to conveniently measure blood pressure whenever blood pressure measurement is necessary, ensures blood flow of the ulnar artery while the blood pressure measurement is performed by pressing a wrist so as to improve accuracy of blood pressure measurement, and is thus applied to a field of medical device manufacturing, particularly, a field of sphygmomanometers.

## Claims

1. A wrist blood pressure monitor wearable on a wrist, comprising:
a wrist cuff comprising a wrist strap to be worn on the wrist; and
a blood pressure measurement sensor provided on the wrist cuff,
wherein the wrist strap comprises:
a strap frame arranged around the ulna in a circumferential direction of the wrist and is bent in a predetermined shape to cross a portion of the wrist through which the ulnar artery passes, so as to maintain a shape of a skeleton of the strap frame; and
a connection strap provided on the strap frame and connecting one end and the other end of the strap frame.

2. The wrist blood pressure monitor according to claim 1, wherein the wrist cuff further comprises an air bag provided on the wrist strap to press a portion of the wrist through which the radial artery passes.

3. The wrist blood pressure monitor according to claim 2, wherein a valve is connected to the air bag so as to enable air supply.

4. The wrist blood pressure monitor according to claim 3, wherein the valve is a check valve configured to be opened due to a pressure difference between an air pressure inside the air bag and atmospheric pressure outside the air bag, so as to implement self-air supply of the air bag.

5. The wrist blood pressure monitor according to claim 2, wherein the blood pressure measurement sensor is provided at the air bag.

6. The wrist blood pressure monitor according to claim 2, wherein the air bag is provided on the connection strap so as to press the portion of the wrist through which the radial artery passes.

7. The wrist blood pressure monitor according to claim 1, wherein the connection strap extends from one end of the strap frame and is detachably connected to the other end of the strap frame.

8. The wrist blood pressure monitor according to claim 7, wherein the connection strap comprises:
a main strap provided with an air bag to press the wrist, and extending from one end of the strap frame; and
an assistant strap extending from the main strap and detachably connected to the other end of the strap frame.

9. The wrist blood pressure monitor according to any one of claims 1 to 8, wherein the strap frame comprises:
a base frame configured to support the connection strap; and
an assistant frame movably provided on the base frame, so as to adjust a height of the wrist cuff corresponding to a wrist thickness.

10. The wrist blood pressure monitor according to any one of claims 1 to 8, wherein the wrist cuff has closed loop shape being continuously connected.
